# EUROPEAN PATENT APPLICATION

(11) **EP 4 585 202 A1**
(43) Date of publication of application: **16.07.2025**
(21) Application number: 23862768.1
(22) Date of filing: 30.06.2023
(51) Int. Cl.: A61K 6/30, A61K 6/60, A61K 6/70

(54) **CARIOUS TOOTH STRUCTURE RECOVERY AGENT, AND DENTAL ADHESIVE KIT**

(30) Priority: 06.09.2022 JP 2022141776
(71) Applicant: Kuraray Noritake Dental Inc., Kurashiki-shi, Okayama 710-0801 (JP)
(72) Inventor: KAWASHIMA, Mitsunobu, Tainai-shi, Niigata 959-2653 (JP)
(74) Representative: D Young & Co LLP
(86) International application number: PCT/JP2023/024376
(87) International publication number: WO 2024/053223

(57) **Abstract**

To provide a carious tooth structure recovery agent that has convenience to treat multiple teeth at once, has aesthetics without discoloration of the treated area, and is capable of performing the preventive care for root surface caries and the initial caries treatment, and the carious tooth structure recovery agent contains a compound (a) having an acidic group represented by the following formula (I): R¹-X (I) (in which R¹ represents an organic group having 40 or less carbon atoms that has or does not have at least one (meth)acryloyloxy group, and X represents an acidic group), a zinc ion (b1), water (c), and a hydrophilic solvent (d), and substantially does not contain a crosslinkable polymerizable monomer.

## Description

### Technical Field

The present invention relates to a carious tooth structure recovery agent and a dental adhesive kit, and more specifically relates to a novel and useful carious tooth structure recovery agent and a dental adhesive kit using the same.

### Background Art

In recent years, the so-called "8020 Campaign", which encourages people to keep 20 or more of their own teeth even at age 80, is leading to an increase in the number of remaining teeth of the elderly. However, the remaining teeth of the elderly are at risk of gingival recession due to periodontal disease or the like, resulting in exposure of the tooth root. The exposed root surface is susceptible to caries. Accordingly, the increase in the number of remaining teeth of the elderly results in the problem of root surface caries, which is specific to the elderly, due to the exposure of the tooth root. Therefore, combined with the increase in the elderly population, the treatment of the root surface caries has become an urgent issue.

The currently available treatment methods widely used for the tooth root caries include filling and restoration with a composite resin or glass ionomer cement, as similar to the treatment of crown caries. However, in the case where the caries progresses below the subgingival margin, it becomes extremely difficult to remove the caries and furthermore to perform the filling operation. The caries may extend over the entire circumference of the tooth root, and the caries makes restoration thereof even more difficult.

The root surface caries often occurs over multiple teeth at once, and the restoration thereof generally requires a long period of time. Accordingly, there are significantly large physical and mental burdens incurred not only by the dental practitioner administering the treatment but also by the patient.

A diamine silver fluoride preparation has been confirmed to be effective from the standpoint of the suppression of the dental caries progression and the prevention of the dental caries occurrence. The diamine silver fluoride preparation is a material that can be easily used since what is required thereby is only to apply the liquid preparation to the affected area, but the application area turns black, and there is room for improvement in aesthetics.

Among the aforementioned problems, from the standpoint of the enhancement of the treatment effectiveness in the filling and restoration with a composite resin, NPLs 1 and 2 describe a photocurable dental adhesive composition containing a compound having a phosphoric acid group, a zinc compound, water, a hydrophilic solvent, a crosslinkable polymerizable monomer, and a polymerization initiator. NPL 1 intends to impart an antibacterial function to the dental adhesive, and NPL 2 intends to enhance the adhesion strength function of the dental adhesive.

PTL 1 describes a treatment agent for hyperesthesia containing a water soluble phosphoric acid compound and water insoluble non-calcium based powder at a prescribed weight ratio, PTL 2 describes a dentifrice composition containing prescribed poorly water soluble powder, a prescribed desensitizing agent, and water, PTL 3 describes an oral composition containing a nonionic surfactant and a prescribed alcohol compound, PTL 4 describes an oral care composition containing a carrier usable in the oral cavity having contained therein a water insoluble zinc compound and a compound having a C-O-P bond, and a PTL 5 describes a tooth surface treatment agent containing a carboxylic acid, zinc chloride, and water.

However, the inventions described in NPLs 1 and 2 and PTLs 1 to 5 fail to describe the restoration of caries to the state close to the healthy tooth structure, and do not consider the treatment of initial caries.

### Citation List

### Patent Literatures

PTL 1: JP 06-092860 A
PTL 2: JP 2013-001648 A
PTL 3: JP 2002-047157 A
PTL 4: JP 2009-520829 A
PTL 5: JP 01-090108 A

### Non-patent Literatures

NPL 1: Journal of the Mechanical Behavior of Biomedical Materials, 100 (2019) 103366
NPL 2: Al-Azhar Dental Journal for Girls, 4 (3) 289-296

### Summary of Invention

### Technical Problem

As described above, the root surface caries is a dental disease that has no suitable treatment method established therefor, and a novel material that is suitable for the disease has been demanded.

In view of the aforementioned problems, a problem to be solved by the present invention is to provide a carious tooth structure recovery agent that has convenience to treat multiple teeth at once, is excellent in aesthetics without discoloration of the treated area, and is capable of performing the preventive care for root surface caries and the initial caries treatment.

### Solution to Problem

As a result of the earnest investigation for solving the problem by the present inventors, it has been newly found that the application of a particular composition containing a compound having an acidic group, a zinc compound, water, and a hydrophilic solvent to the tooth surface strengthens not only the demineralized tooth structure but also the healthy tooth structure in a short period of time. Furthermore, it has also been found that the tooth surface having the particular composition coated thereon can acquire caries resistance, and after further cumulative investigation, the present invention has been completed.

Specifically, the present invention encompasses the following inventions.
[1] A carious tooth structure recovery agent containing
   a compound (a) having an acidic group represented by the following formula (I):

      R¹-X (I)

      in which R¹ represents an organic group having 40 or less carbon atoms that has or does not have at least one (meth)acryloyloxy group, and X represents an acidic group,
   a zinc ion (b1), water (c), and a hydrophilic solvent (d),
   substantially not containing a crosslinkable polymerizable monomer.
[2] The carious tooth structure recovery agent according to claim 1, in which the compound (a) having an acidic group is represented by the following formula (II):

   R¹-((X^{m-})ₐ·(H⁺)_{b})ₖ (II)

   in which R¹ has the same meaning as defined in the formula (I), X^{m-} represents an m-valent acid anion, m represents an integer of 1, 2, or 3, a and b represent integers of 1, 2, or 3 that have a relationship of am = b, and k represents an integer of 1 or 2.
[3] The carious tooth structure recovery agent according to the item [1] or [2], in which the compound (a) having an acidic group is represented by the following formula (III):

   R²-R³-O-P(=O)(-OH)₂ (III)

   in which R² represents a (meth)acryloyloxy group or hydrogen, and R³ represents an alkylene group having 6 to 20 carbon atoms.
[4] The carious tooth structure recovery agent according to any one of the items [1] to [3], in which a content of the compound (a) having an acidic group is 10 to 70% by mass based on the total mass as 100% by mass of the compound (a) having an acidic group, the water (c), and the hydrophilic solvent (d).
[5] The carious tooth structure recovery agent according to any one of the items [1] to [4], wherein the zinc ion (b1) is derived from at least one zinc compound (b) selected from the group consisting of ZnO and a compound represented by the following formula (IV):

   (Zn²⁺)_{c}·(Yⁿ⁻)_{d} (IV)

   in which Yⁿ⁻ represents an anion as a counter ion of the zinc ion, n represents an integer of 1, 2, or 3, and c and d each represent an integer of 1, 2, or 3, in which c, d, and n have a relationship of 2c = nd.
[6] The carious tooth structure recovery agent according to any one of the items [1] to [5], in which the zinc ion (b1) is derived from at least one zinc compound (b) selected from the group consisting of ZnO, ZnCl₂, Zn(OH)₂, and ZnF₂.
[7] The carious tooth structure recovery agent according to any one of the items [1] to [6], in which a content of the zinc compound (b) is 0.3 to 10% by mass based on the total mass as 100% by mass of the compound (a) having an acidic group, the water (c), and the hydrophilic solvent (d).
[8] The carious tooth structure recovery agent according to any one of the items [1] to [7], in which the carious tooth structure recovery agent contains an organic compound other than the compound (a) having an acidic group and the hydrophilic solvent (d) in an amount of 5 parts by mass or less per 100 parts by mass of the carious tooth structure recovery agent.
[9] A dental adhesive kit including the carious tooth structure recovery agent [A] according to any one of the items [1] to [8], and a dental adhesive [B].

### Advantageous Effects of Invention

The present invention can provide a carious tooth structure recovery agent that can further strengthen the healthy tooth structure, and can restore the demineralized and weakened tooth structure due to caries to the strength close to the healthy tooth structure, while securing high aesthetics, through a convenient operation of applying to the tooth surface.

### Description of Embodiments

In the description herein, the "strength" in stating that the "tooth structure is strong" means the mechanical strength and the resistance to demineralization caused by an acid.

Further, in the description herein, the upper limit values and the lower limit values of the numeral range (such as the content of the component, the value calculated from the components, and the property value) can be appropriately combined.

Furthermore, in the description herein, the "carious tooth structure recovery agent" means a dental material that is applied to a demineralized and weakened tooth structure due to caries, and thereby restores the tooth structure close to the healthy tooth structure, and is a dental material that restores at least an initial demineralized tooth structure in initial caries. The "carious tooth structure recovery agent" may further have a function of strengthening the tooth structure and a function of preventing caries of the tooth structure.

### [Carious Tooth Structure Recovery Agent]

The carious tooth structure recovery agent according to an embodiment of the present invention contains
a compound (a) having an acidic group represented by the following formula (I):

   R¹-X (I)

   in which R¹ represents an organic group having 40 or less carbon atoms that has or does not have at least one (meth)acryloyloxy group, and X represents an acidic group,
a zinc ion (b1), water (c), and a hydrophilic solvent (d), and
substantially does not contain a crosslinkable polymerizable monomer.

In the description herein, the "(meth)acryloyloxy" is a generic term for methacryloyloxy and acryloyloxy, which is also applied to the similar terms.

The present inventors estimate the mechanism that the carious tooth structure recovery agent provides the effects of the present invention as follows while not limiting thereto.

By applying the carious tooth structure recovery agent to a tooth structure surface, a deposit is formed on the entire coated tooth surface. It is considered that the deposit is a substance formed in such a manner that the acidic mixture formed of the compound (a) having an acidic group, the water (c), and the hydrophilic solvent (d) demineralizes the smear layer of the surface layer of the tooth structure and the surface layer of the tooth structure and penetrates into the tooth structure, and in this process, the compound (a) having an acidic group, the zinc ion (b1), and the inorganic component of the demineralized tooth structure compound to be precipitated and deposited on the tooth structure surface, and it is estimated that the formation thereof enhances the mechanical strength of the tooth structure, and also imparts a caries resisting effect thereto.

In general, caries with parenchymal defect requires restoration of the crown morphology with a restoration material. In this case, the use of the carious tooth structure recovery agent can restore the demineralized and weakened tooth structure due to caries to the strength close to the healthy tooth structure, and thereby the adhesion of the restoration material to the tooth structure can be further stabilized, achieving the restoration with high reliability.

In the description herein, the function of enhancing the mechanical strength of the healthy tooth structure, the function of enhancing the mechanical strength of the carious dentin, and the function of enhancing the resistance of the healthy tooth structure against demineralization with an acid, which are provided by the carious tooth structure recovery agent, may be collectively referred to as "tooth structure strengthening".

The compositions described in NPLs 1 and 2 also contain a compound having an acidic group, a hydrophilic solvent, water, and a zinc compound. However, the compositions described in NPLs 1 and 2 are designed to further contain a crosslinkable polymerizable monomer in a significant amount for exerting the function of a dental adhesive, which is the intended purpose thereof, through firm hardening with a photopolymerization initiator.

Accordingly, the compositions described in NPLs 1 and 2 are a photopolymerization type dental adhesive. The dental adhesive is an acidic mixture, and it has been known that the dental adhesive applied to the tooth surface demineralizes the smear layer of the surface layer of the tooth structure and the surface layer of the tooth structure, and penetrates into the tooth structure. On the other hand, NPLs 1 and 2 do not describe the formation of a deposit on the tooth surface by applying the compositions described in NPLs 1 and 2 to the tooth structure surface, and also do not describe the strengthening of the tooth structure achieved by applying the compositions described in NPLs 1 and 2 to the tooth surface.

In the compositions described in NPLs 1 and 2, it is estimated that the presence of the crosslinkable polymerizable monomer, which is one kind of an organic compound mixed in addition to the compound (a) having an acidic group and the hydrophilic solvent (d), inhibits the function of compounding the compound having an acidic group, the zinc compound, and the inorganic component of the demineralized tooth structure, or inhibits the necessary amount of the deposit from being formed. As a result, in NPLs 1 and 2, it is estimated that no deposit is formed on the tooth structure surface, failing to exert the function of strengthening the tooth structure.

### <Compound (a) having Acidic Group>

The compound (a) having an acidic group represented by the formula (I) is a compound having an acidic group that allows the acidic composition obtained by mixing the water (c) and the hydrophilic solvent (d) to exert the acid etching effect and the primer treatment effect to the tooth structure, and is a component that imparts the demineralization function and the penetration function.

The compound (a) having an acidic group represented by the formula (I) is preferably represented by the following formula (II):

R¹-((X^{m-})ₐ·(H⁺)_{b})ₖ (II)

in which R¹ has the same meaning as defined in the formula (I), X^{m-} represents an m-valent acid anion, m represents an integer of 1, 2, or 3, a and b represent integers of 1, 2, or 3 that have a relationship of am = b, and k represents an integer of 1 or 2.

Examples of the compound (a) having an acidic group include a compound having at least one acidic group, such as a phosphoric acid group, a pyrophosphoric acid group, a thiophosphoric acid group, a phosphonic acid group, a sulfonic acid group, and a carboxylic acid group. Specific examples of the compound (a) having an acidic group are shown below.

Examples of the phosphoric acid group-containing compound include octyl dihydrogen phosphate, decyl dihydrogen phosphate, dodecyl dihydrogen phosphate, tetradecyl dihydrogen phosphate, hexadecyl dihydrogen phosphate, octadecyl dihydrogen phosphate, 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl] hydrogen phosphate, bis[4-(meth)acryloyloxybutyl] hydrogen phosphate, bis[6-(meth)acryloyloxyhexyl] hydrogen phosphate, bis[8-(meth)acryloyloxyoctyl] hydrogen phosphate, bis[9-(meth)acryloyloxynonyl] hydrogen phosphate, bis[10-(meth)acryloyloxydecyl] hydrogen phosphate, 1,3-di(meth)acryloyloxypropyl dihydrogen phosphate, 2-(meth)acryloyloxyethylphenyl hydrogen phosphate, 2-(meth)acryloyloxyethyl-2-bromoethyl hydrogen phosphate, and bis[2-(meth)acryloyloxy-(1-hydroxymethyl)ethyl] hydrogen phosphate.

Examples of the pyrophosphoric acid group-containing compound include bis[2-(meth)acryloyloxyethyl] pyrophosphate, bis[4-(meth)acryloyloxybutyl] pyrophosphate, bis[6-(meth)acryloyloxyhexyl] pyrophosphate, bis[8-(meth)acryloyloxyoctyl] pyrophosphate, and bis[10-(meth)acryloyloxydecyl] pyrophosphate.

Examples of the thiophosphoric acid group-containing compound include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, and 20-(meth)acryloyloxyicosyl dihydrogen thiophosphate.

Examples of the phosphonic acid group-containing compound include 2-(meth)acryloyloxyethyl phenylphosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl phosphonoacetate, and 10-(meth)acryloyloxydecyl phosphonoacetate.

Examples of the sulfonic acid group-containing compound include 2-(meth)acrylamido-2-methylpropanesulfonic acid, styrenesulfonic acid, and 2-sulfoethyl (meth)acrylate.

Examples of the carboxylic acid group-containing compound include a compound having one carboxy group in a molecule and a compound having multiple carboxy groups in a molecule.

Examples of the compound having one carboxy group in a molecule include (meth)acrylic acid, N-(meth)acryloylglycine, N-(meth)acryloylaspartic acid, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, p-vinylbenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, 2-(meth)acryloyloxyethyl hydrogen succinate, 2-(meth)acryloyloxyethyl hydrogen phthalate, and 2-(meth)acryloyloxyethyl hydrogen maleate.

Examples of the compound having multiple carboxy groups in a molecule include 6-(meth)acryloyloxyhexane-1,1-dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1,1-dicarboxylic acid, 12-(meth)acryloyloxydodecane-1,1-dicarboxylic acid, 13-(meth)acryloyloxytridecane-1,1-dicarboxylic acid, 4-(meth)acryloyloxyethyl trimellitate, 4-(meth)acryloyloxybutyl trimellitate, 4-(meth)acryloyloxyhexyl trimellitate, 4-(meth)acryloyloxydecyl trimellitate, and 2-(meth)acryloyloxyethyl-3'-(meth)acryloyloxy-2'-(3,4-dicarboxybenzoyloxy)propyl succinate.

The compound (a) having an acidic group may be a compound represented by the following formula (III):

R²-R³-O-P(=O)(-OH)₂ (III)

in which R² represents a (meth)acryloyloxy group or hydrogen, and R³ represents an alkylene group having 6 to 20 carbon atoms.

Among the compound (a) having an acidic group, a phosphoric acid group-containing or pyrophosphoric acid group-containing compound is preferred since a better deposit formation effect is exerted thereby on the tooth structure, and a phosphoric acid group-containing compound is particularly preferred. Among these, as exerting the high tooth structure demineralization and the high deposit formation, a divalent phosphoric acid group-containing compound having an alkyl group having 6 to 20 carbon atoms as a main chain in a molecule, or a divalent phosphoric acid group-containing compound having an alkylene group having 6 to 20 carbon atoms as a main chain in a molecule, such as the compound represented by the formula (III), are more preferred, and a divalent phosphoric acid group-containing compound having an alkyl or alkylene group having 8 to 12 carbon atoms as a main chain in a molecule, such as dodecyl dihydrogen phosphate and 10-(meth)acryloyloxydecyl dihydrogen phosphate, is most preferred.

One kind of the compound (a) having an acidic group may be used alone, or two or more kinds thereof may be used in combination. The content of the compound (a) having an acidic group is preferably 10% by mass or more, more preferably 15% by mass or more, and further preferably 20% by mass or more, based on the total mass of the compound (a) having an acidic group, the water (c), and the hydrophilic solvent (d) in the carious tooth structure recovery agent as 100% by mass, from the standpoint of facilitating the securement of the effects of the present invention. The content thereof is preferably 70% by mass or less, and more preferably 60% by mass or less. In other words, the content of the compound (a) having an acidic group is preferably 10 to 70% by mass based on the total mass of the compound (a) having an acidic group, the water (c), and the hydrophilic solvent (d) as 100% by mass.

### <Zinc Ion (b1) and Zinc Compound (b)>

The zinc ion (b1) is derived from the zinc compound (b). The zinc compound (b) is dissolved with the compound (a) having an acidic group, so as to allow the carious tooth structure recovery agent to contain the zinc ion (b1).

The zinc ion (b1) is preferably derived from at least one zinc compound (b) selected from the group consisting of ZnO and a compound represented by the following formula (IV):

(Zn²⁺)_{c}·(Yⁿ⁻)_{d} (IV)

in which Yⁿ⁻ represents an anion as a counter ion of the zinc ion, n represents an integer of 1, 2, or 3, and c and d each represent an integer of 1, 2, or 3, in which c, d, and n have a relationship of 2c = nd.

Specific examples of the zinc compound (b) include at least one zinc compound (b) selected from ZnO, ZnCl₂, Zn(OH)₂, and ZnF₂, in which ZnO is preferred.

One kind of the zinc compound (b) may be used alone in the preparation of the carious tooth structure recovery agent, or two or more kinds thereof may be used in combination. Accordingly, one kind of zinc ion (b1) may exist alone in the carious tooth structure recovery agent, or two or more kinds thereof may exist therein.

The amount of the zinc compound (b) mixed in the carious tooth structure recovery agent is preferably 0.3% by mass or more, more preferably 0.6% by mass or more, and further preferably 1.0% by mass or more, from the standpoint of facilitating the favorable formation of the deposit for strengthening the tooth structure, and is preferably 10% by mass or less, more preferably 7.0% by mass or less, and further preferably 5.0% by mass or less, from the standpoint of suppressing the neutralization of the acidic group of the compound (a) having an acidic group from proceeding excessively, and securing the favorable demineralization function and penetration function, all based on the total mass (100% by mass) of the compound (a) having an acidic group, the water (c), and the hydrophilic solvent (d). In other words, the content of the zinc compound (b) is preferably 0.3 to 10% by mass based on the total mass as 100% by mass of the compound (a) having an acidic group, the water (c), and the hydrophilic solvent (d).

The content of the zinc ion (b1) in the carious tooth structure recovery agent is preferably 0.1% by mass or more, more preferably 0.2% by mass or more, and further preferably 0.3% by mass or more, and is preferably 8.0% by mass or less, more preferably 5.6% by mass or less, and further preferably 4.0% by mass or less, all based on the total mass (100% by mass) of the compound (a) having an acidic group, the water (c), and the hydrophilic solvent (d) , from the same standpoints. In other words, the content of the zinc ion (b1) is preferably 0.1 to 8.0% by mass based on the total mass as 100% by mass of the compound (a) having an acidic group, the water (c), and the hydrophilic solvent (d).

The content of the zinc ion (b1) in the carious tooth structure recovery agent can be calculated based on the mixing amount of the zinc compound (b), or after determining the content of the zinc ion (b1) through measurement with an ICP emission spectrometer, can be calculated therefrom.

### <Water (c)>

The water (c) preferably does not contain impurities that cause adverse effects, and is preferably distilled water or ion exchanged water. The content of the water (c) in the carious tooth structure recovery agent is preferably 1 to 90% by mass, more preferably 5 to 80% by mass, and further preferably 10 to 70% by mass, based on the total mass as 100% by mass of the compound (a) having an acidic group, the water (c), and the hydrophilic solvent (d).

### <Hydrophilic Solvent (d)>

The hydrophilic solvent (d) means an organic compound that has a solubility of 10% by mass or more in water at 25°C, and is preferably one having the solubility that is 30% by mass or more, and more preferably one that can be dissolved in water in any ratio at 25°C.

The hydrophilic solvent (d) may be a single compound or a mixture of multiple compounds.

Examples of the hydrophilic solvent (d) include ethanol, 2-propanol, 2-methyl-2-propanol, acetone, tetrahydrofuran, 2-hydroxyethyl (meth)acrylate, 3-hydroxypropyl (meth)acrylate, 2-hydroxypropyl (meth)acrylate, 1,3-dihydroxypropyl (meth)acrylate, 2,3-dihydroxypropyl (meth)acrylate, 2-((meth)acryloyloxy)ethyl trimethylammonium chloride, polyethylene glycol di(meth)acrylate (in which the number of oxyethylene groups is 9 or more), N,N-dimethylaminoethyl methacrylate, N-methylol (meth)acrylamide, N-hydroxyethyl (meth)acrylamide, N,N-bis(2-hydroxyethyl) (meth)acrylamide, N-methoxymethyl (meth)acrylamide, N-ethoxymethyl (meth)acrylamide, diacetone (meth)acrylamide, 4-(meth)acryloylmorpholine, N-trihydroxymethyl-N-methyl (meth)acrylamide, N-[3-(dimethylamino)propyl]acrylamide, and a (meth)acrylamide represented by the following formula (V): 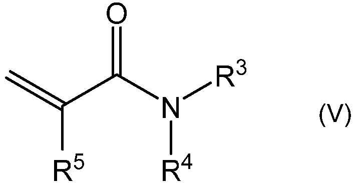 in which R³ and R⁴ each independently represent an alkyl group having 1 to 3 carbon atoms (such as a methyl group, an ethyl group, a n-propyl group, and an isopropyl group), which may have a substituent (such as a hydroxy group), and R⁵ represents a hydrogen atom or a methyl group.

In the hydrophilic solvent (d), ethanol, 2-propanol, 2-methyl-2-propanol, acetone, tetrahydrofuran, 2-hydroxyethyl (meth)acrylate, 2,3-dihydroxypropyl (meth)acrylate, N,N-dimethylaminoethyl methacrylate, diacetone (meth)acrylamide, and a hydrophilic monofunctional (meth)acrylamide based monomer are preferred, and ethanol, 2-propanol, 2-hydroxyethyl (meth)acrylate, N,N-dimethylaminoethyl methacrylate, and a monofunctional (meth)acrylamide based monomer represented by the formula (V) are more preferred. One kind of the hydrophilic solvent (d) may be mixed alone, or two or more kinds thereof may be used in combination.

The hydrophilic solvent (d) functions as a cosolvent of the compound (a) having an acidic group and the water (c). The content of the hydrophilic solvent (d) in the carious tooth structure recovery agent is preferably 1 to 90% by mass, more preferably 5 to 80% by mass, and further preferably 10 to 70% by mass, based on the total mass as 100% by mass of the compound (a) having an acidic group, the water (c), and the hydrophilic solvent (d).

### <Organic Compound other than Compound (a) having Acidic Group and Hydrophilic Solvent (d)>

The carious tooth structure recovery agent substantially does not contain a crosslinkable polymerizable monomer, which is an organic compound other than the compound (a) having an acidic group and the hydrophilic solvent (d). The expression "substantially not containing" herein means that the amount of the crosslinkable polymerizable monomer is 5 parts by mass or less, and preferably 3 parts by mass or less, per 100 parts by mass of the carious tooth structure recovery agent.

Examples of the crosslinkable polymerizable monomer include an aromatic bifunctional monomer, an aliphatic bifunctional monomer, and a trifunctional or higher functional monomer, which are hydrophobic crosslinkable polymerizable monomers having no acidic group.

The carious tooth structure recovery agent according to an embodiment of the present invention substantially does not contain does not contain a crosslinkable polymerizable monomer, which thereby prevents the function of compounding the compound (a) having an acidic group, the zinc ion (b1), and the inorganic component of the demineralized tooth structure from being inhibited, accelerating the formation of the deposit thereby.

Examples of the aromatic bifunctional monomer include 2,2-bis((meth)acryloyloxyphenyl)propane, 2,2-bis[4-(3-(meth)acryloyloxy-2-hydroxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane.

Examples of the aliphatic bifunctional monomer include glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1,2-bis(3-methacryloyloxy-2-hydroxypropoxy)ethane, 2,2,4-trimethylhexamethylene bis(2-carbamoyloxy)ethyl di(meth)acrylate, N-methacryloyloxyethyl acrylamide, N-methacryloyloxypropyl acrylamide, N-methacryloyloxybutyl acrylamide, N-(1-ethyl-(2-methacryloyloxy)ethyl) acrylamide, and N-(2-(2-methacryloyloxyethoxy)ethyl) acrylamide.

Examples of the trifunctional or higher functional monomer include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol] tetra(meth)acrylate, and 1,7-diacryloyloxy-2,2,6,6-tetra(meth)acryloyloxymethyl-4-oxyheptane.

The carious tooth structure recovery agent preferably substantially does not contain an organic compound other than the compound (a) having an acidic group and the hydrophilic solvent (d), which includes the crosslinkable polymerizable monomer above. The expression "substantially not containing" herein means that the amount of the organic compound other than the compound (a) having an acidic group and the hydrophilic solvent (d) is 5 parts by mass or less, and preferably 3 parts by mass or less, per 100 parts by mass of the carious tooth structure recovery agent.

Examples of the organic compound other than the compound (a) having an acidic group and the hydrophilic solvent (d) and other than the crosslinkable polymerizable monomer include:
a photopolymerization initiator, such as a (bis)acylphosphine oxide compound, a thioxanthone compound or a quaternary ammonium salt of a thioxanthone compound, a ketal compound, an α-diketone compound, a coumarin compound, an anthraquinone compound, a benzoyl alkyl ether compound, and an α-aminoketone based compound;
a polymerization accelerator, such as an aldehyde compound, a thiol compound, and an aminobenzoate ester compound;
an antibacterial substance, such as cetylpyridinium chloride, benzalkonium chloride, (meth)acryloyloxyldecylpyridinium bromide, (meth)acryloyloxyhexadecylpyridinium chloride, (meth)acryloyloxydecylammonium chloride, triclosan, and 12-methacryloyloxydodecylpyridinium bromide; and
a monofunctional polymerizable monomer, such as 4-hydroxybutyl (meth)acrylate, 6-hydroxyhexyl (meth)acrylate, 8-hydroxyoctyl (meth)acrylate, 10-hydroxydecyl (meth)acrylate, and octafluoropentyl (meth)acrylate.

Specific examples of the (bis)acylphosphine oxide compound used as the polymerization initiator include 2,4,6-trimethylbenzoyl diphenylphosphine oxide, 2,4,6-trimethylbenzoyl methoxyphenylphosphine oxide, bis(2,4,6-trimethylbenzoyl)phenylphosphine oxide, and sodium 2,4,6-trimethylbenzoyl phenylphosphine oxide.

Specific examples of the α-diketone compound used as the polymerization initiator include diacetyl, benzil, camphorquinone, 2,3-pentanedione, 2,3-octanedione, 9,10-phenanthrenequinone, 4,4'-oxybenzyl, and acenaphthenequinone.

Specific examples of the aldehyde compound used as the polymerization accelerator include terephthalaldehyde and a benzaldehyde derivative. Examples of the benzaldehyde derivative include dimethylaminobenzaldehyde, p-methoxybenzaldehyde, p-ethoxybenzaldehyde, and p-n-octyloxybenzaldehyde.

Specific examples of the thiol compound used as the polymerization accelerator include 2-mercaptobenzoxazole, decanethiol, 3-mercaptopropyltrimethoxysilane, and thiobenzoic acid.

Specific examples of the aminobenzoate ester compound used as the polymerization accelerator include ethyl 4-(N,N-dimethylamino)benzoate, methyl 4-(N,N-dimethylamino)benzoate, n-butoxyethyl 4-N,N-(dimethylamino)benzoate, 2-[(meth)acryloyloxy]ethyl 4-(N,N-dimethylamino)benzoate, 4-(N,N-dimethylamino)benzophenone, and n-butyl 4-(N,N-dimethylamino)benzoate.

### <Additional Component>

The carious tooth structure recovery agent may further contain one kind or two or more kinds of additional components. Examples of the additional component include a pH modifier, a polymerization inhibitor, an ultraviolet ray absorbent, a thickener, a colorant (such as a dye and a pigment), a fluorescent agent, and a perfume. Further, the carious tooth structure recovery agent may contain, for example, as the additional component, a fluoride ion releasing material, such as sodium fluoride, potassium fluoride, sodium monofluorophosphate, lithium fluoride, strontium fluoride, and ytterbium fluoride. Furthermore, the carious tooth structure recovery agent may contain, for example, a metal compound, such as calcium hydroxide, calcium chloride, silver oxide, and aluminum hydroxide.

The amount of the additional component mixed is not particularly limited, and may be such an amount that can provide the necessary effect, and the amount thereof is preferably 0.01 to 1 part by mass, more preferably 0.05 to 0.5 part by mass, and further preferably 0.07 to 0.3 part by mass, per 100 parts by mass of the carious tooth structure recovery agent, from the standpoint of facilitating the sufficient securement of the effectiveness of the strengthening of the tooth structure and the treatment of initial caries.

However, in the case where the additional component is an organic compound, the amount thereof mixed is 5 parts by mass or less, and more preferably 3 parts by mass or less, per 100 parts by mass of the carious tooth structure recovery agent.

The carious tooth structure recovery agent according to one embodiment of the present invention may contain a calcium ion as the additional component, as long as exerting the effects of the present invention, but preferably substantially does not contain a calcium ion from the standpoint of further facilitating the effects of the present invention.

In the case where the carious tooth structure recovery agent substantially does not contain a calcium ion, it is estimated that the influence of a calcium ion on at least one function in the formation process of the deposit caused by the carious tooth structure recovery agent of the function of precipitating the compound (a) having an acidic group, the zinc ion (b1), and the inorganic component of the demineralized tooth structure through compounding, and the function of depositing on the tooth structure surface, and thereby the deposit can be more easily formed thereon.

The calcium ion is derived from a calcium compound. The calcium compound is dissolved with the compound (a) having an acidic group, so as to allow the carious tooth structure recovery agent to contain the calcium ion.

The calcium ion is derived, for example, from at least one calcium compound selected from the group consisting of CaO and a compound represented by the following formula (VI):

(Ca²⁺)ₑ·(Y^{p-})_{f} (VI)

in which Yⁿ⁻ represents an anion as a counter ion of the calcium ion, p represents an integer of 1, 2, or 3, e and f each represent an integer of 1, 2, or 3, in which e, f, and p have a relationship of 2e = pf.

More specific examples of the calcium compound include at least one calcium compound selected from the group consisting of CaO, CaCl₂, Ca(OH)₂, and CaF₂.

The expression "substantially does not contain" a calcium ion herein means that the content of the calcium ion is 5% by mass or less, preferably 3% by mass or less, more preferably 1% by mass or less, further preferably 0.1% by mass or less, and still further preferably 0.05% by mass or less, based on the carious tooth structure recovery agent as 100% by mass. In other words, the expression "substantially does not contain a calcium ion" in the description herein means that the content of the calcium ion is 0 to 5% by mass, preferably 0 to 3% by mass, more preferably 0 to 1% by mass, further preferably 0 to 0.1% by mass, and still further preferably 0 to 0.05% by mass, based on the carious tooth structure recovery agent as 100% by mass.

In the description herein, the content of the calcium ion in the carious tooth structure recovery agent can be calculated based on the mixing amount of the calcium compound forming the calcium ion, or can also be obtained through measurement with an ICP emission spectrometer.

### [Method for Producing Carious Tooth Structure Recovery Agent]

The method for producing the carious tooth structure recovery agent according to an embodiment of the present invention may include adding the compound (a) having an acidic group, the zinc compound (b), the water (c), and the hydrophilic solvent (d) in any order, and the carious tooth structure recovery agent can be obtained by providing finally the state where the zinc compound (b) as a solid component is dissolved in the liquid mixture composition containing the compound (a) having an acidic group, the water (c), and the hydrophilic solvent (d).

### [Application of Carious Tooth Structure Recovery Agent]

The carious tooth structure recovery agent can be delivered as a one-component type liquid, and can be applied to a tooth structure with a swab, a sponge chip, a small brush, an applicator brush, or the like. After applying, the volatile component may be dried through air blowing with a three-way syringe or the like depending on necessity, so as to complete the treatment. Due to the convenient operative procedure, the stress experienced by both the practitioner and the patient is minimal even in treating multiple teeth.

The tooth structure strengthening function provided by the carious tooth structure recovery agent according to an embodiment of the present invention is particularly significant for dentin, and therefore the carious tooth structure recovery agent can be favorably used as a "dentin strengthening agent".

The carious tooth structure recovery agent according to an embodiment of the present invention can be favorably applied to the purposes of the prevention of occurrence of caries, the suppression of progression of initial caries, the suppression of secondary caries, and the suppression of hyperesthesia of dentin.

### [Dental Adhesive Kit]

The dental adhesive kit according to an embodiment of the present invention includes a carious tooth structure recovery agent [A] and a dental adhesive [B], in which the carious tooth structure recovery agent described above is used as the carious tooth structure recovery agent [A].

The dental adhesive [B] is not particularly limited, and various dental adhesives having been known as a dental bonding material can be used. The dental adhesive [B] is generally an adhesive composition containing an acidic component and water, and may further contain a polymerizable monomer, a polymerization initiator, a solvent, and other admixtures depending on necessity. Examples of the particularly preferred dental adhesive [B] include a dental bonding material containing an acidic group-containing (meth)acrylic based polymerizable monomer, a water soluble polymerizable monomer, and a polymerization initiator, as described in WO 2013/145621 and WO 2015/190099. The dental adhesive [B] may be a one-component type capable of being used directly, or may be a two-component type including two components, a component A and a component B, which are mixed immediately before use.

Examples of the commercially available product of the dental adhesive [B] include a dental bonding material "Clearfil (registered trade name) Universal Bond Quick ER", available from Kuraray Noritake Dental Inc.

In the dental adhesive kit, the carious tooth structure recovery agent [A] contains the components (a) to (d) and substantially does not contain an organic compound other than the components (a) and (d), and thereby the application thereof to the treatment area of a tooth provides the strengthening of the tooth structure and the restoration of the initial demineralized tooth structure. After the application, the dental adhesive [B] is fed to the applied surface of the carious tooth structure recovery agent [A], and thereby a filling material, such as a composite resin, a coating material, or the like further fed thereto can be firmly adhered, resulting in favorable restoration of the tooth. In this operation, at least the carious tooth structure recovery agent [A] can be used through application, a convenient treatment, and high aesthetics can also be secured.

The present invention encompasses embodiments including various combinations of the aforementioned configurations within the scope of the technical concept of the present invention, as long as exerting the effects of the present invention.

### Examples

The carious tooth structure recovery agent according to the present invention and the dental adhesive kit according to the present invention will be specifically described with reference to examples below, but the carious tooth structure recovery agent and the dental adhesive kit according to the present invention are not limited to the examples.

### [Preparation of Tensile Test Specimen of Dentin (Healthy Dentin)]

The labial surface of a bovine mandibular anterior tooth was polished with #80 silicon carbide polishing paper (available from Nihon Kenshi Co., Ltd.) under running water to expose the flat surface of dentin. Subsequently, the lingual surface thereof was polished in the same manner as above to provide dentin in the form of plate. The resulting dentin in the form of plate was polished with #1000 silicon carbide polishing paper (available from Nihon Kenshi Co., Ltd.) under running water to a thickness of 1 mm. Subsequently, the dentin having a thickness of 1 mm was cut into a width of 1 mm with an automatic precision saw, IsoMet (available from Buehler Ltd.), to provide a healthy dentin specimen having a rectangular columnar shape of 1 mm in thickness × 1 mm in width × approximately 15 mm in length.

### [Preparation of Tensile Test Specimen of Dentin (Caries Model Dentin)]

The healthy dentin specimen shaped into a rectangular columnar shape of 1 mm in thickness × 1 mm in width × approximately 15 mm in length was immersed in a 0.5 M acetic acid buffer solution (pH 4.6) at 37°C for 24 hours, so as to produce a caries model dentin specimen having a rectangular columnar shape of 1 mm in thickness × 1 mm in width × approximately 15 mm in length.

### [Measurement of Tensile Strength of Healthy Dentin and Caries Model Dentin]

The healthy and caries model dentin specimens having a rectangular columnar shape of 1 mm in thickness × 1 mm in width × approximately 15 mm in length each were fixed to a jig for a microscale tensile test with a cyanoacrylate based adhesive. The tensile strength of the dentin was measured with a compact desktop universal testing machine (available from Shimadzu Corporation) under condition of a crosshead speed of 1 mm/min. Five specimens were measured for each of the conditions, and the average value was designated as the tensile strength.

The tensile strength of the healthy dentin was 109 MPa, and the tensile strength of the caries model dentin was 44 MPa.

The abbreviations of the components used in Examples and Comparative Examples are as follows.

### [Compound (a) having Acidic Group]

MHP: 6-methacryloyloxyhexyl dihydrogen phosphate
MDP: 10-methacryloyloxydecyl dihydrogen phosphate
M12P: 12-methacryloyloxydodecyl dihydrogen phosphate
4-MET: 4-methacryloyloxyethyl trimellitate
M4P: 4-methacryloyloxybutyl dihydrogen phosphate

### [Hydrophilic Solvent (d)]

HEMA: 2-hydroxyethyl methacrylate
DEAA: N,N-diethylacrylamide (compound represented by the formula (V))

### [Organic Compound other than Compound (a) having Acidic Group and Hydrophilic Solvent (d)]

CPC: cetylpyridinium chloride
Bis-GMA: 2,2-bis[4-(3-methacryloyloxy-2-hydroxypropoxy)phenyl]propane
CQ: DL-camphorquinone
EDMB: ethyl 4-(N,N-dimethylamino)benzoate

### [Preparation and Evaluation of Carious Tooth Structure Recovery Agent]

### <Examples 1 to 11 and Comparative Examples 1 to 4>

The materials were mixed according to the formulations shown in Table 1 to produce carious tooth structure recovery agents of Examples and Comparative Examples. A specimen was produced with each of the compositions in the following manner, and the specimen was measured according to the following procedure. In Comparative Example 1, the composition caused phase separation, failing to measure the tensile strength.

### (Tensile Strength [X] of Healthy Dentin after Treatment)

Each of the compositions was applied to the healthy dentin specimen having a rectangular columnar shape of 1 mm in thickness × 1 mm in width × approximately 15 mm in length with an applicator brush, and immediately subjected to an air blow treatment. The specimen was fixed to a jig for a microscale tensile test with a cyanoacrylate based adhesive. The tensile strength of the dentin was measured with a compact desktop universal testing machine (available from Shimadzu Corporation) under condition of a crosshead speed of 1 mm/min. Five specimens were measured for each of the conditions, and the average value was designated as the tensile strength [X] of the healthy dentin after the treatment.

### (Tensile Strength [Y] of Healthy Dentin after Treatment followed by Artificial Caries Treatment)

Each of the compositions was applied to the healthy dentin specimen having a rectangular columnar shape of 1 mm in thickness × 1 mm in width × approximately 15 mm in length with an applicator brush, and immediately subjected to an air blow treatment. Thereafter, the specimens were immersed in a 0.5 M acetic acid buffer solution (pH 4.6) at 37°C for 24 hours, so as to prepare an artificial caries treatment group. The specimen was fixed to a jig for a microscale tensile test with a cyanoacrylate based adhesive. The tensile strength of the dentin was measured with a compact desktop universal testing machine (available from Shimadzu Corporation) under condition of a crosshead speed of 1 mm/min. Five specimens were measured for each of the conditions, and the average value was designated as the tensile strength [Y] of the healthy dentin after the treatment followed by the artificial caries treatment.

### (Tensile Strength [Z] of Caries Model Dentin after Treatment)

Each of the compositions was applied to the caries model dentin specimen having a rectangular columnar shape of 1 mm in thickness × 1 mm in width × approximately 15 mm in length with an applicator brush, and immediately subjected to an air blow treatment. The specimen was fixed to a jig for a microscale tensile test with a cyanoacrylate based adhesive. The tensile strength of the dentin was measured with a compact desktop universal testing machine (available from Shimadzu Corporation) under condition of a crosshead speed of 1 mm/min. Five specimens were measured for each of the conditions, and the average value was designated as the tensile strength [Z] of the caries model dentin after the treatment.

The results of the tensile strength [X] of the healthy dentin after the treatment, the tensile strength [Y] of the healthy dentin after the treatment followed by the artificial caries treatment, and the tensile strength [Z] of the caries model dentin after the treatment are shown in Table 1. The ratios of [X] and the ratios of [Y] with respect to the tensile strength of the healthy dentin (109 MPa) are shown in Table 1. The ratios of [Z] with respect to the tensile strength of the caries model dentin (44 MPa) are shown in Table 1.

**Table 1**

| | Name of component | Unit | Example 1 | Example 2 | Example 3 | Example 4 | Example 5 | Example 6 | Example 7 | Example 8 |
|---|---|---|---|---|---|---|---|---|---|---|
| Compound (a) having acidic group | Dodecyl phosphate | part by mass | 30 | - | - | - | - | - | - | - |
| | Hexadecyl phosphate | part by mass | - | 30 | - | - | - | - | 30 | 30 |
| | MHP | part by mass | - | - | 30 | - | - | - | - | - |
| | MDP | part by mass | - | - | - | 30 | - | 30 | - | - |
| | M12P | part by mass | - | - | - | - | 30 | - | - | - |
| | 4-MET | part by mass | - | - | - | - | - | - | - | - |
| | M4P | part by mass | - | - | - | - | - | - | - | - |
| Zinc compound (b) | ZnO | part by mass | 1.7 | 1.5 | 1.7 | 1.5 | 1.4 | 1.5 | - | - |
| | ZnCl₂ | part by mass | - | - | - | - | - | - | 3.8 | - |
| | Zn(OH)₂ | part by mass | - | - | - | - | - | - | - | 2 |
| Water (c) | Water | part by mass | 35 | 35 | 35 | 35 | 35 | 35 | 35 | 35 |
| Hydrophilic solvent (d) | Ethanol | part by mass | 35 | 35 | 35 | - | 35 | - | - | 35 |
| | Isopropanol | part by mass | - | - | - | 35 | - | - | - | - |
| | HEMA | part by mass | - | - | - | - | - | 35 | - | - |
| | DEAA | part by mass | - | - | - | - | - | - | 35 | - |
| Additional component | NaF | part by mass | - | - | - | - | - | - | 0.1 | - |
| | Ca(OH)₂ | part by mass | - | - | - | - | - | - | - | 0.1 |
| | CaCl₂ | part by mass | - | - | - | - | - | - | - | - |
| | Ag₂O | part by mass | - | - | - | - | - | - | - | - |
| Organic compound other than components (a) and (d) | CPC | part by mass | - | - | - | - | - | - | - | - |
| | Bis-GMA | part by mass | - | - | - | - | - | - | - | - |
| | CQ | part by mass | - | - | - | - | - | - | - | - |
| | EDMB | part by mass | - | - | - | - | - | - | - | - |
| Tensile strength [X] of healthy dentin after treatment | | MPa | 133 | 130 | 127 | 148 | 135 | 164 | 122 | 120 |
| Tensile strength [Y] healthy dentin after treatment followed by artificial caries treatment | | MPa | 110 | 109 | 107 | 111 | 108 | 116 | 102 | 98 |
| Tensile strength [Z] of caries model dentin after treatment | | MPa | 94 | 92 | 88 | 96 | 93 | 97 | 84 | 79 |
| Ratio of [X] to tensile strength of untreated healthy dentin (109 MPa) | | | 1.22 | 1.19 | 1.17 | 1.36 | 1.24 | 1.50 | 1.12 | 1.10 |
| Ratio of [Y] to tensile strength of untreated healthy dentin (109 MPa) | | | 1.01 | 1.00 | 0.98 | 1.02 | 0.99 | 1.06 | 0.94 | 0.90 |
| Ratio of [Z] to tensile strength of caries model dentin (44 MPa) | | | 2.1 | 2.1 | 2.0 | 2.2 | 2.1 | 2.2 | 1.9 | 1.8 |

**Table 1 (continued)**

| | Name of component | Unit | Example 9 | Example 10 | Example 11 | Comparative Example 1 | Comparative Example 2 | Comparative Example 3 | Comparative Example 4 |
|---|---|---|---|---|---|---|---|---|---|
| Compound (a) having acidic group | Dodecyl phosphate | part by mass | - | - | - | - | - | - | - |
| | Hexadecyl phosphate | part by mass | - | - | - | - | - | 26.6 | - |
| | MHP | part by mass | - | - | - | - | - | - | 30 |
| | MDP | part by mass | - | - | - | 30 | 10 | - | - |
| | M12P | part by mass | - | 28 | - | - | - | - | - |
| | 4-MET | part by mass | 30 | - | - | - | - | - | - |
| | M4P | part by mass | - | - | 30 | - | - | - | - |
| Zinc compound (b) | ZnO | part by mass | 1.5 | 1.6 | 1.6 | 1.5 | 1.0 | 1.4 | - |
| | ZnCl₂ | part by mass | - | - | - | - | - | - | - |
| | Zn(OH)₂ | part by mass | - | - | - | - | - | - | - |
| Water (c) | Water | part by mass | 35 | 32.7 | 35 | 70 | 14 | 31 | 30 |
| Hydrophilic solvent (d) | Ethanol | part by mass | 35 | 32.7 | 35 | - | 14 | 31 | |
| | Isopropanol | part by mass | - | - | - | - | - | - | 35 |
| | HEMA | part by mass | - | - | - | - | 28 | - | - |
| | DEAA | part by mass | - | - | - | - | - | - | - |
| Additional component | NaF | part by mass | - | - | - | - | - | - | - |
| | Ca(OH)₂ | part by mass | - | - | - | - | - | - | - |
| | CaCl₂ | part by mass | 0.1 | - | - | - | - | - | - |
| | Ag₂O | part by mass | - | - | - | - | - | - | 4.2 |
| Organic compound other than components (a) and (d) | CPC | part by mass | - | 5.0 | - | - | - | - | - |
| | Bis-GMA | part by mass | - | - | - | - | 30 | 10 | - |
| | CQ | part by mass | - | - | - | - | 2.0 | - | - |
| | EDMB | part by mass | - | - | - | - | 1.0 | - | - |
| Tensile strength [X] of healthy dentin after treatment | | MPa | 115 | 119 | 114 | unable to test due to phase separation of composition | 110 | 113 | 92 |
| Tensile strength [Y] healthy dentin after treatment followed by artificial caries treatment | | MPa | 75 | 87 | 67 | | 48 | 65 | 52 |
| Tensile strength [Z] of caries model dentin after treatment | | MPa | 66 | 70 | 65 | | 47 | 64 | 42 |
| Ratio of [X] to tensile strength of untreated healthy dentin (109 MPa) | | | 1.06 | 1.09 | 1.05 | | 1.01 | 1.04 | 0.84 |
| Ratio of [Y] to tensile strength of untreated healthy dentin (109 MPa) | | | 0.69 | 0.80 | 0.61 | | 0.44 | 0.60 | 0.48 |
| Ratio of [Z] to tensile strength of caries model dentin (44 MPa) | | | 1.5 | 1.6 | 1.5 | | 1.1 | 1.5 | 1.0 |

It is understood from the results in Table 1 that the treatment with the carious tooth structure recovery agent containing the components (a) to (d) and substantially not containing an organic compound other than the components (a) and (d) has the function of enhancing the tensile strength of the healthy dentin. It is also understood that the healthy dentin treated with the carious tooth structure recovery agent containing the components (a) to (d) and substantially not containing an organic compound other than the components (a) and (d) retains the strength thereof even subjecting to the artificial caries treatment, and acquires caries resistance. It is further understood that the treatment of the caries model dentin having a decreased tensile strength due to the artificial caries treatment with the carious tooth structure recovery agent containing the components (a) to (d) and substantially not containing an organic compound other than the components (a) and (d) can restore the tensile strength.

The carious tooth structure recovery agents of Examples 1 to 8 using the compound represented by the formula (III) as the compound (a) have larger values for the "ratio of the tensile strength of the healthy dentin after the treatment and the tensile strength of the untreated healthy dentin", the "ratio of the tensile strength of the healthy dentin after the treatment followed by the artificial caries treatment and the tensile strength of the untreated healthy dentin", and the "ratio of the tensile strength of the caries model dentin after the treatment and the tensile strength of the caries model dentin" than the carious tooth structure recovery agents of Examples 9 and 11 using the compound (a) that does not correspond to the formula (III), and the carious tooth structure recovery agent of Example 10 using the compound represented by the formula (III) as the compound (a) but containing the organic compound other than the compound (a) and the hydrophilic solvent (d).

On the other hand, it is understood from the results of Comparative Example 2 containing the organic compound other than the compound (a) having an acidic group and the hydrophilic solvent (d) in an amount of 33 parts by mass per 100 parts by mass of the composition, prepared according to the compositions used in NPLs 1 and 2, and Comparative Example 3 prepared by adding 10 parts by mass of the organic compound other than the compound (a) having an acidic group and the hydrophilic solvent (d) to 90 parts by mass of the composition of Example 2 that the treatment with a composition containing an organic compound other than the compound (a) having an acidic group and the hydrophilic solvent (d) in an amount of larger than 5 parts by mass per 100 parts by mass of the composition fails to show the function of enhancing the tensile strength of the healthy dentin. It is also understood that the carious tooth structure recovery agents of Comparative Examples 2 and 3 do not acquire resistance to the artificial caries treatment, and do not show the restoration of the tensile strength of the caries model dentin having a decreased tensile strength due to the artificial caries treatment.

It is understood from the result of Comparative Example 4 that the treatment with the composition obtained by replacing the zinc compound (b) with the other metal compound does not show the function of enhancing the tensile strength of the healthy dentin. It is also understood that the carious tooth structure recovery agent of Comparative Example 4 does not acquire resistance to the artificial caries treatment, and does not show the restoration of the tensile strength of the caries model dentin having a decreased tensile strength due to the artificial caries treatment.

### [Combination Use of Carious Tooth Structure Recovery Agent and Dental Adhesive]

### <Example 12>

Assuming the treatment of applying the carious tooth structure recovery agent to the tooth surface in filling and restoration of a caries cavity, an adhesion test to artificial carious dentin was performed. Specifically, the labial surface of a bovine mandibular anterior tooth was polished with #80 silicon carbide polishing paper (available from Nihon Kenshi Co., Ltd.) under running water to prepare a specimen having an exposed flat surface of dentin. The resulting specimen was further polished with #1000 silicon carbide polishing paper (available from Nihon Kenshi Co., Ltd.) under running water. After polishing, water on the surface thereof was dried through air blowing.

Subsequently, the tooth surface was coated with a nail varnish except for a center part (4 mm × 4 mm) of the exposed surface of dentin, and then the dentin was immersed in 0.5 M acetic acid buffer solution (pH 4.6) at 37°C for 24 hours to form caries model dentin on the exposed surface of dentin. The caries model dentin prepared in the manner described above was rinsed under running water, and water on the surface thereof was dried through air blowing. To the flat surface after drying (i.e., the part having the caries model dentin formed therein), an adhesive tape having a thickness of approximately 150 µm having a circular hole having a diameter of 3 mm was attached to define the adhesion area.

The carious tooth structure recovery agent produced in Example 1 was applied inside the circular hole with an applicator brush, and then immediately the surface thereof was dried through air blowing. Subsequently, a dental bonding material (available from Kuraray Noritake Dental Inc., product name "Clearfil (registered trade name) Universal Bond Quick ER"), was applied inside the circular hole with an applicator brush, and after rubbing for 10 seconds, the surface thereof was dried through air blowing until the applied dental bonding material lost the fluidity. Subsequently, the applied dental bonding material was cured through light irradiation for 3 seconds with a dental LED light irradiator (available from Morita Corporation, product name "Pencure 2000").

On the surface of the resulting cured product of the dental bonding material, a dental filling composite resin (available from Kuraray Noritake Dental Inc., product name "Clearfil (registered trade name) AP-X") was filled, which was then covered with a release film (polyester). Then, the applied surface of the dental filling composite resin was flattened by pressing slide glass onto the release film. Subsequently, the dental filling composite resin was cured by irradiating the dental filling composite resin with light for 20 seconds with a dental LED light irradiator (available from Morita Corporation, product name "Pencure 2000").

On the surface of the resulting cured product of the dental filling composite resin, one end surface (circular cross section) of a stainless steel circular column (diameter: 7 mm, length: 2.5 cm) was adhered with a commercially available dental resin cement (available from Kuraray Noritake Dental Inc., product name "Panavia (registered trade name) 21"). After adhering, the specimen was allowed to stand at room temperature for 30 minutes, and then immersed in distilled water, so as to provide an adhesion test specimen. Five adhesion test specimens were produced, and allowed to stand in a thermostat chamber retained to 37°C for 24 hours in the state of immersing in distilled water.

The tensile strength was measured by using a universal testing machine (available from Shimadzu Corporation) at a crosshead speed of 2 mm/min. The arithmetic average value of the resulting values was designated as the tensile adhesion strength. The tensile adhesion strength was 19 MPa. The result is shown in Table 2.

### <Comparative Example 5>

The same operation as in Example 12 was performed except that the operation of "applying the carious tooth structure recovery agent produced in Example 1 inside the circular hole with an applicator brush, and then immediately drying the surface thereof through air blowing" in Example 12 was omitted, and the tensile strength of the dental bonding material to the caries model dentin was measured. The tensile adhesion strength was 9 MPa. The result is shown in Table 2.

**Table 2**

| | Unit | Example 12 | Comparative Example 5 |
|---|---|---|---|
| Carious tooth structure recovery agent [A] | - | Dental composition of Example 1 | none |
| Dental adhesive [B] | - | Quick ER | Quick ER |
| Tensile strength after treating caries model dentin | MPa | 19 | 9 |

It is understood from the results of Example 12 and Comparative Example 5 shown in Table 2 that the tensile adhesion strength of the dental bonding material to the caries model dentin treated with the carious tooth structure recovery agent containing the components (a) to (d) and substantially not containing an organic compound other than the components (a) and (d) is advantageously larger than the tensile strength thereof to the untreated caries model dentin.

### [Influence on Color Tone of Tooth Structure]

### <Example 13>

The composition of Example 1 was applied to the tooth root of a bovine mandibular anterior tooth with an applicator brush, and subjected to air blowing. The tooth was immersed in distilled water and stored at 37°C for 24 hours, and then the visual observation of the applied part revealed that no change in color tone was found. The result is shown in Table 3.

### <Comparative Example 6>

A diamine silver fluoride preparation "Saforide Dental Solution 38%" (available from Bee Brand Medico Dental Co., Ltd.) was applied to the tooth root of a bovine mandibular anterior according to the usage and dosage instructions described in the prescribing information. The tooth was immersed in distilled water and stored at 37°C for 24 hours, and then the visual observation of the applied part revealed that an obvious change in color tone to brown color was found. The result is shown in Table 3.

**Table 3**

| | Example 13 | Comparative Example 6 |
|---|---|---|
| Carious tooth structure recovery agent | Carious tooth structure recovery agent of Example 1 | Diamine silver fluoride preparation |
| Change in color tone | none | found |

As apparent from the comparison between Example 13 and Comparative Example 6 shown in Table 3, the carious tooth structure recovery agent containing the components (a) to (d) and substantially not containing an organic compound other than the components (a) and (d) is free of change in color tone to black or brown color, which occurs in the applied area of the diamine silver fluoride preparation, and can secure high aesthetics.

### Industrial Applicability

The carious tooth structure recovery agent of the present invention can achieve the strengthening of a tooth structure and the restoration of an initial demineralized tooth structure through a convenient operation while securing high aesthetics, and therefore can be favorably applied particularly to the preventive care for root surface caries and the restoration treatment of initial caries.

## Claims

1. A carious tooth structure recovery agent comprising
a compound (a) having an acidic group represented by the following formula (I):
R¹-X (I)
wherein R¹ represents an organic group having 40 or less carbon atoms that has or does not have at least one (meth)acryloyloxy group, and X represents an acidic group,
a zinc ion (b1), water (c), and a hydrophilic solvent (d),
substantially not comprising a crosslinkable polymerizable monomer.

2. The carious tooth structure recovery agent according to claim 1, wherein the compound (a) having an acidic group is represented by the following formula (II):
R¹-((X^{m-})ₐ·(H⁺)_{b})ₖ (II)
wherein R¹ has the same meaning as defined in the formula (I), X^{m-} represents an m-valent acid anion, m represents an integer of 1, 2, or 3, a and b represent integers of 1, 2, or 3 that have a relationship of am = b, and k represents an integer of 1 or 2.

3. The carious tooth structure recovery agent according to claim 1 or 2, wherein the compound (a) having an acidic group is represented by the following formula (III):
R²-R³-O-P(=O)(-OH)₂ (III)
wherein R² represents a (meth)acryloyloxy group or hydrogen, and R³ represents an alkylene group having 6 to 20 carbon atoms.

4. The carious tooth structure recovery agent according to any one of claims 1 to 3, wherein a content of the compound (a) having an acidic group is 10 to 70% by mass based on the total mass as 100% by mass of the compound (a) having an acidic group, the water (c), and the hydrophilic solvent (d).

5. The carious tooth structure recovery agent according to any one of claims 1 to 4, wherein the zinc ion (b1) is derived from at least one zinc compound (b) selected from the group consisting of ZnO and a compound represented by the following formula (IV):
(Zn²⁺)_{c}·(Yⁿ⁻)_{d} (IV)
wherein Yⁿ⁻ represents an anion as a counter ion of the zinc ion, n represents an integer of 1, 2, or 3, and c and d each represent an integer of 1, 2, or 3, in which c, d, and n have a relationship of 2c = nd.

6. The carious tooth structure recovery agent according to any one of claims 1 to 5, wherein the zinc ion (b1) is derived from at least one zinc compound (b) selected from the group consisting of ZnO, ZnCl₂, Zn(OH)₂, and ZnF₂.

7. The carious tooth structure recovery agent according to any one of claims 1 to 6, wherein a content of the zinc compound (b) is 0.3 to 10% by mass based on the total mass as 100% by mass of the compound (a) having an acidic group, the water (c), and the hydrophilic solvent (d).

8. The carious tooth structure recovery agent according to any one of claims 1 to 7, wherein the carious tooth structure recovery agent comprises an organic compound other than the compound (a) having an acidic group and the hydrophilic solvent (d) in an amount of 5 parts by mass or less per 100 parts by mass of the carious tooth structure recovery agent.

9. A dental adhesive kit comprising the carious tooth structure recovery agent [A] according to any one of claims 1 to 8, and a dental adhesive [B].
